# EUROPEAN PATENT SPECIFICATION

(11) **EP 1 838 362 B1**
(45) Date of publication and mention of the grant of the patent: **14.05.2014**
(21) Application number: 05853392.8
(22) Date of filing: 08.12.2005
(51) Int. Cl.: A61L 27/50, A61L 29/16, A61L 31/14

(54) **USE OF SUPERCRITICAL FLUIDS TO INCORPORATE BIOLOGICALLY ACTIVE AGENTS INTO NANOPOROUS MEDICAL ARTICLES**
VERWENDUNG ÜBERKRITISCHER FLÜSSIGKEITEN ZUM INKORPORIEREN VON BIOLOGISCH AKTIVEN WIRKSTOFFEN IN NANOPORÖSE MEDIZINISCHE ARTIKEL
UTILISATION DE FLUIDES SUPERCRITIQUES POUR INCORPORER DES AGENTS BIOLOGIQUEMENT ACTIFS DANS DES ARTICLES MEDICAUX NANOPOREUX

(30) Priority: 09.12.2004 US 7866
(43) Date of publication of application: 03.10.2007
(73) Proprietor: Boston Scientific Limited, Hastings Christ Church (BB)
(72) Inventor: HELMUS, Michael, N., Worcester, MA 01609 (US)
(74) Representative: Vossius & Partner
(86) International application number: PCT/US2005/044462
(87) International publication number: WO 2006/063158

(56) References cited:
- WO-A-94/18264
- US-A1- 2003 044 514
- GERCKENS ULRICH ET AL: "Evaluation of a Tacrolimus-eluting coronary stent with nanoporous ceramic coating in treatment of native coronary artery lesions: Phase I and II of the present study." JOURNAL OF THE AMERICAN COLLEGE OF CARDIOLOGY, vol. 41, no. 6 Supplement A, 19 March 2003 (2003-03-19), page 7A, XP002381361 & 52ND ANNUAL SCIENTIFIC SESSION OF THE AMERICAN COLLEGE OF CARDIOLOGY; CHICAGO, IL, USA; MARCH 30-APRIL 02, 2003 ISSN: 0735-1097

## Description

### TECHNICAL FIELD

This invention relates to medical devices containing biologically active agents, and in particular to methods of loading medical articles with biologically active agents.

### BACKGROUND

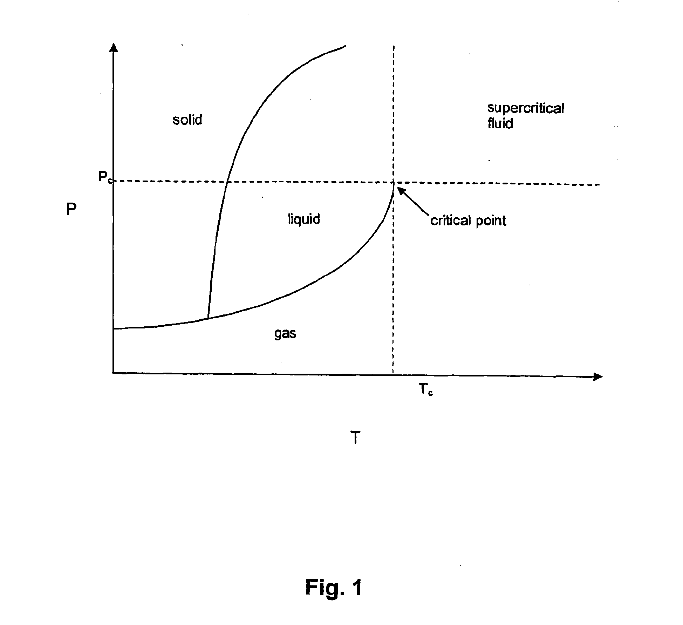

A supercritical fluid is a substance that has been subjected to conditions that are above the critical temperature and critical pressure of that substance. This range of conditions is illustrated in the generalized schematic phase diagram of Fig. 1. The supercritical region is the range of conditions that are found in the upper right-hand portion of Fig. 1, where the temperature is above the critical temperature (T_{c}) and the pressure is above the critical pressure (P_{c}). This combination of critical temperature and pressure is known as the critical point. Hence, stated another way, a substance becomes a supercritical where its temperature and pressure are above its critical point (i.e., T>T_{c} and P>P_{c}) Various non-supercritical phase transitions between solid and liquid (melting), between liquid and gas (boiling), and between solid and gas (sublimation) are also illustrated in Fig. 1.

A supercritical fluid exhibits both gas-like and liquid-like properties. The density of the supercritical fluid may be similar to that of a very dense gas and its diffusivity may be similar to diffusivities normally associated with gases, while its solubility properties may be similar to that of a liquid. Hence, a fluid in the supercritical state is sometimes described as having the behavior of a very mobile liquid, in which the solubility behavior approaches that of the liquid phase while penetration into a solid matrix is facilitated by the gas-like transport properties. Supercritical fluids will exhibit these properties as long as they are maintained in their supercritical range. However, when either the temperature or the pressure of a supercritical fluid drops below its associated critical point, the fluid is no longer classified as a supercritical fluid, because it no longer posses some or all of the mixed property characteristics associated with a substance in this range.

Supercritical fluids are used to extract various components from a wide variety of materials in a process commonly known as supercritical extraction. In some cases, the solubility of various components in a supercritical fluid is enhanced by the addition of a substance known as a cosolvent. The volatility of this additional component is usually intermediate that of the supercritical fluid and the substance to be extracted and/or to be imbibed (see below).

Supercritical fluids have also been used in imbibing medical devices with therapeutic agents. See, e.g., U.S. Patent Application No. 20030044514 entitled "Using supercritical fluids to infuse therapeutic on a medical device" naming Robert E. Richard as an inventor.

### SUMMARY OF THE INVENTION

Nanoporous materials are known in the medical field. For example, U.S. Patent Application 20020042657 entitled "Synthetic biomaterial compound of calcium phosphate phases particularly adapted for supporting bone cell activity" describes a nanoporous synthetic biomaterial compound based on stabilized calcium phosphates. As another example nanoporous silica xerogels are described in Radin et al., "In vitro bioactivity and degradation behavior of silica xerogels intended as controlled release materials," Biomaterials 23 (2002) 3113-3122. Other nanoporous materials, including various nanoporous metals, polymers and ceramics are also known.

In many instances, it is desirable to incorporate biologically active agents into medical articles such as implantable or insertable medical devices. For instance, in the case of coronary stents, it is frequently desirable to incorporate anti-restenotic therapeutic agents into the same for controlled release.

Nanoporous material offer the benefit of very high surface areas for biologically active agent disposal. Moreover, providing materials with nanoscale features such as nanopores has been observed to have a marked effect upon the interactions between those materials and surrounding tissues or cells.

While biologically active agents may be incorporated into nanoporous structures during the formation of the same, in many instances this is not possible, for example, due to processing conditions that would result in the deactivation or destruction of the biologically active agent, if present.

These and other drawbacks are overcome by the present invention in which a supercritical fluid that comprises a carrier fluid and a biologically active agent is brought into contact with a medical article that comprises a nanoporous surface region according to claim 1.

For example, the nanoporous surface region of the medical article can be contacted with a biologically active agent dissolved in supercritical carbon dioxide.

Examples of biologically active agents include anti-restenotic agents such as paclitaxel, and agents that promote tissue adhesion, such as glycosaminoglycans, proteoglycans, adhesion peptides, and adhesive proteins, among many others.

The nanoporous surface region is, for example, metallic, ceramic, polymeric or a combination thereof. For instance, in certain embodiments, the nanoporous surface region comprises a metal, for example, a noble metal or a metal alloy. In certain embodiments, the nanoporous surface region comprises a metal oxide, for example, an aluminum oxide, a silicon oxide, an alkaline earth metal oxides or a transition metal oxide. In certain embodiments, the nanoporous surface region comprises a bioactive material, for example, a bioactive metal oxide, such as aluminum oxide or titanium oxide, or hydroxyapatite.

**A variety of tubular medical articles may be used in the practice of the present invention.** In certain beneficial embodiments, the medical article is an implantable or insertable medical device, for example, a bone plate, a joint prosthesis, a vascular graft, a stent graft, a stent, a catheter, a guide wire, a balloon, a filter, a vascular patch, a shunt, or a coil.

The use supercritical fluids to load nanoporous surface regions of medical articles with biologically active agents is advantageous, for example, because supercritical fluids can be used to solubilize a wide variety of biologically active agents. Moreover, the low viscosities and surface energies associated with supercritical fluids promote entry into small pores, as compared with subcritical solvents (including water), which have significantly higher viscosities and surface energies.

Supercritical fluids are also advantageous in that they are highly compressible, allowing the quantity of the biologically active agent that is introduced into the nanopores to be increased with increasing pressure.

These and other embodiments and advantages of the present invention will become immediately apparent to those of ordinary skill in the art upon review of the Detailed Description and Claims to follow.

### BRIEF DESCRIPTION OF THE DRAWINGS

Fig. 1 is a generalized schematic phase diagram of a hypothetical substance, illustrating the supercritical range of conditions for the substance.
Fig. 2 is a flow diagram illustrating a process in which stents having a nanoporous surface region are exposed to a supercritical mixture of carbon dioxide and a drug, according to an embodiment of the present invention.
Fig. 3 is a flow diagram illustrating a process in which stents having a nanoporous surface region are exposed to a supercritical mixture of carbon dioxide and a drug, according to another embodiment of the present invention.

### DETAILED DESCRIPTION

The present invention is directed to processes for loading medical articles with biologically active agents. In accordance with an embodiment of the present invention, a medical article according to claim 1 that comprises a nanoporous surface region, is contacted with a supercritical fluid that comprises a carrier fluid and a biologically active agent. As a result of this contact, biologically active agent is transferred from the supercritical fluid to the nanoporous surface of the medical device.

A nanoporous surface region is one that comprises a plurality of nanopores (commonly at least 10⁶, 10⁹, 10¹² or more nanopores per cm²). A "nanopore," as the term is used herein, is a surface concavity, indentation, opening or orifice, at least one lateral dimension of which (e.g., the diameter for a cylindrical pore, the length or width for a non-cylindrical pore, etc.) does not exceed 100 nm. A nanopore typically, although not necessarily, has a depth that is greater than its largest lateral surface. Moreover, the surfaces will typically, although not necessarily, further comprise pores that are not nanopores (e.g., pores that are larger than nanopores). For example, in some embodiments, up to 20% by number of the pores may be larger than nanopores.

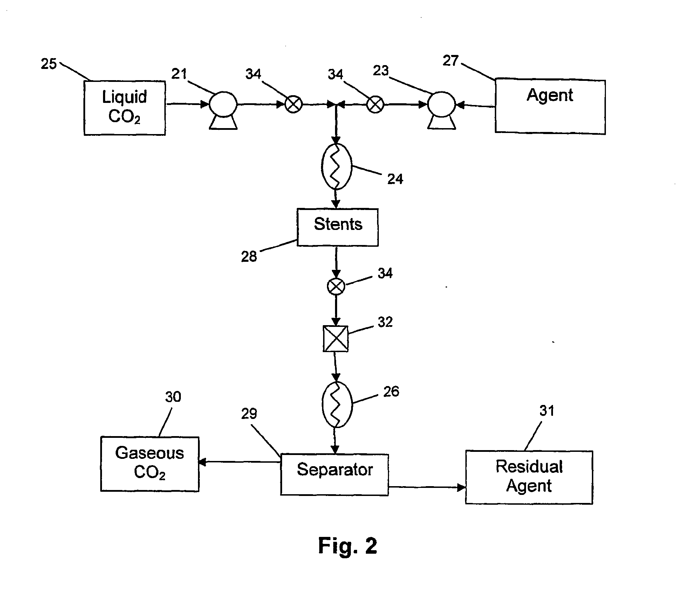

One specific embodiment of the present invention is illustrated in conjunction with Fig. 2, in which stents having nanoporous surface regions are loaded with a drug using CO₂ as a carrier fluid. Referring now to Fig. 2, a source of CO₂ 25, in this case liquid CO₂, is provided. The liquid CO₂ from source 25 passed through pump 21, to a region having a pressure that is above the critical pressure of the liquid CO₂.

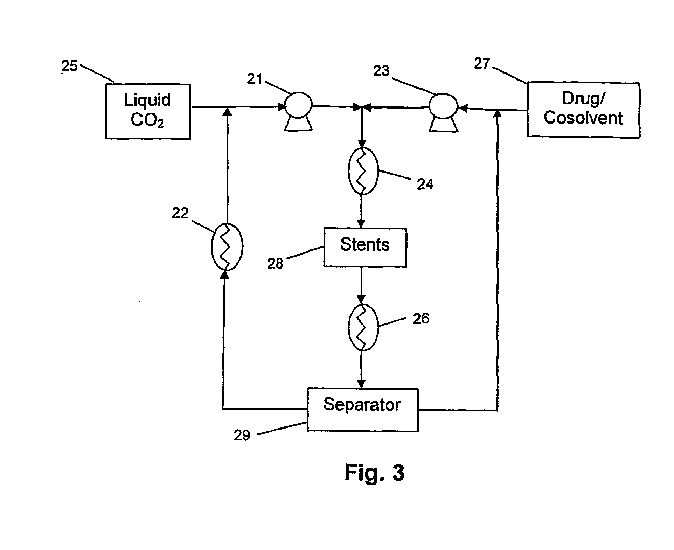

The CO₂ stream is joined by a stream of biologically active agent from source 27, which is pumped to the same pressure as the CO₂ stream via pump 23. If desired, the biologically active agent can be dissolved or provided as a colloidal suspension in a cosolvent as is illustrated in Fig. 3 below. In other embodiments, it can be dissolved or suspended within the liquid carrier fluid (e.g., dissolved or suspended in liquid CO₂).

Turning again to Fig. 2, the stream containing the CO₂ and biologically active agent, which are above critical pressure at this stage, are heated to a temperature that is above the critical temperature using heater 24. The mixture, at this point in the supercritical realm, is then placed into contact with stents in a chamber 28, whereupon the biologically-active-agent-containing supercritical mixture penetrates the nanoporous surface regions of the stents, for example, due to the gas-like transport properties of the supercritical mixture.

After exposure to the stents 28, the supercritical mixture passes through valve 34, restrictor 32 (e.g., a capillary restrictor or restrictor valve) and evaporator 26, which results in the expansion of the CO₂ into the gas phase and the precipitation of other components such as any residual biologically active agent and any cosolvent, if employed. The gaseous CO₂ is then separated from the other components by passing the mixture through a separator 29 (e.g., trap).

As illustrated in Fig. 3, the CO₂ can be recycled, for example, by passing the gaseous CO₂ through a condenser 22, returning it to liquid form. Similarly, the drug and any associated cosolvent can also be recycled, for example, as illustrated in Fig. 3.

In certain embodiments of the invention, deposition and/or precipitation of the biologically active agent is influenced by controlling the rate at which the carrier fluid is removed from the chamber. For example, deposition and/or precipitation of the biologically active agent may be increased by reducing the rate at which the carrier fluid is bled from the chamber.

Although Figs. 2 and 3 describe an apparatus and process in which supercritical CO₂ is used to load stents with a biologically active agent, other carrier fluids, other medical articles and other apparatuses can obviously be utilized.

For example, carbon dioxide is an attractive choice for use as a supercritical fluid. It is an abundant, non-toxic, non-flammable material that exhibits a high level of solubility when placed in its supercritical range. However, carbon dioxide is but is one example of various substances that placed into its supercritical range. Other commonly used substances include acetylene, ammonia, argon, carbon tetrafluoride, cyclohexane, dichlorodifluoromethane, ethane, ethylene, hydrogen, krypton, methane, neon, nitrogen, nitrous oxide, oxygen, pentane, propane, propylene, toluene, trichlorofluoromethane, trifluoromethane, trifluorochloromethane and xenon, among others.

Moreover, the present invention is applicable to various medical articles besides stents. Medical articles for use in conjunction with the present invention include controlled drug delivery devices and devices that are implanted or inserted into the body, for example, for procedural uses or as implants. Implantable or insertable medical devices for use in conjunction with the present invention include bone plates, joint prostheses, central venous catheters, vascular access ports, cannulae, metal wire ligatures, stents (including coronary vascular stents, cerebral, urethral, ureteral, biliary, tracheal, gastrointestinal and esophageal stents), stent grafts, catheters (for example, renal or vascular catheters such as balloon catheters), guide wires, balloons, filters (e.g., vena cava filters), tissue scaffolding devices, tissue bulking devices, embolization devices including cerebral aneurysm filler coils (e.g., Guglilmi detachable coils and metal coils), vascular grafts, heart valves, left ventricular assist hearts and pumps, total artificial hearts, and biopsy devices.

The medical devices of the present invention may be used for systemic treatment or for localized treatment of any mammalian tissue or organ. Non-limiting examples are tumors; organs including but not limited to the heart, coronary and peripheral vascular system (referred to overall as "the vasculature"), lungs, trachea, esophagus, brain, liver, kidney, bladder, urethra and ureters, eye, intestines, stomach, pancreas, ovary, and prostate; skeletal muscle; smooth muscle; breast; cartilage; and bone.

As used herein, "treatment" refers to the prevention of a disease or condition, the reduction or elimination of symptoms associated with a disease or condition, or the substantial or complete elimination a disease or condition. Preferred subjects (also referred to as "patients") are vertebrate subjects, more preferably mammalian subjects and more preferably human subjects.

The tubular medical devices for use in conjunction with the present invention have a nanoporous surface region on the abluminal surface and the luminal surface, which can be formed over the entire surface of the device or only a portion (or portions) thereof. Moreover, in various embodiments, the nanopores are formed within a coating on the device surface or are formed in the surface of a monolithic device. Hence, one or more nanoporous surface regions are provided on the medical device surface at desired locations and/or in desired shapes (e.g., in desired **patterns) on its abluminal surface and its luminal surface. For example, for tubular devices such as stents (which can comprise, for** example, a laser or mechanically cut tube, one or more braided, woven, or knitted filaments, etc), the nanoporous surface regions are present on the luminal surface and on **the abluminal surface, e.g., patterned on said surfaces along the luminal or abluminal length of the device.**

Moreover, multiple nanoprous regions having the same or different biologically active agents can be provided, for instance, using appropriate masking techniques. As an example, it is possible to provide a tubular tubular medical device (e.g., a vascular stent) having a first nanoporous region comprising a first biologically active agent (e.g., an antithrombotic agent) on its inner luminal, surface and a second nanoporous region comprising a second biologically active agent that differs from the first biologically active agent (e.g., an antiproliferative agent) on its outer, abluminal surface (as well as on the ends).

Materials within which nanopores are formed may comprise ceramic materials. Examples of ceramic materials include silica- and/or calcium-phosphate-based glasses, sometimes referred to as glass ceramics (e.g., silica and bioglass); calcium phosphate ceramics (e.g., hydroxyapatite); metal oxides, including aluminum oxides and transition metal oxides (e.g., oxides of titanium, zirconium, hafnium, tantalum, molybdenum, tungsten, rhenium and iridium); and carbon based ceramic-like materials such as silicon carbides and carbon nitrides.

Several ceramic materials are known to be bioactive in nature. By "bioactive" is meant that these materials promote bonding with adjacent tissue (e.g., bone tissue, vascular tissue, mucosal tissue, soft tissue, and so forth), typically with minimal adverse biological effects (e.g., the formation of unwanted connective tissue, for instance, the formation of a capsule of fibrous connective tissue). Examples of bioactive ceramics include oxides of titanium and aluminum, as well as hydroxyapatite.

Materials within which nanopores are formed may also comprise metals, for example, silver, gold, platinum, palladium, iridium, osmium, rhodium, titanium, tungsten, and ruthenium and metal alloys such as cobalt-chromium alloys, nickel-titanium alloys

(e.g., nitinol), cobalt-chromium-iron alloys (e.g., elgiloy alloys), nickel-chromium alloys (e.g., inconel alloys), and iron-chromium alloys (e.g., stainless steels, which contain at least 50% iron and at least 11.5% chromium).

Materials within which nanopores are formed may also comprise polymers, including one or more of the following: polycarboxylic acid polymers and copolymers including polyacrylic acids; acetal polymers and copolymers; acrylate and methacrylate polymers and copolymers (e.g., n-butyl methacrylate); cellulosic polymers and copolymers, including cellulose acetates, cellulose nitrates, cellulose propionates, cellulose acetate butyrates, cellophanes, rayons, rayon triacetates, and cellulose ethers such as carboxymethyl celluloses and hydoxyalkyl celluloses; polyoxymethylene polymers and copolymers; polyimide polymers and copolymers such as polyether block imides, polyamidimides, polyesterimides, and polyetherimides; polysulfone polymers and copolymers including polyarylsulfones and polyethersulfones; polyamide polymers and copolymers including nylon 6,6, nylon 12, polycaprolactams and polyacrylamides; resins including alkyd resins, phenolic resins, urea resins, melamine resins, epoxy resins, allyl resins and epoxide resins; polycarbonates; polyacrylonitriles; polyvinylpyrrolidones (cross-linked and otherwise); polymers and copolymers of vinyl monomers including polyvinyl alcohols, polyvinyl halides such as polyvinyl chlorides, ethylene-vinylacetate copolymers (EVA), polyvinylidene chlorides, polyvinyl ethers such as polyvinyl methyl ethers, polystyrenes, styrene-maleic anhydride copolymers, styrene-butadiene copolymers, styrene-ethylene-butylene copolymers (e.g., a polystyrene-polyethylene/butylene-polystyrene (SEBS) copolymer, available as Kraton® G series polymers), styrene-isoprene copolymers (e.g., polystyrene-polyisoprene-polystyrene), acrylonitrile-styrene copolymers, acrylonitrile-butadiene-styrene copolymers, styrene-butadiene copolymers and styrene-isobutylene copolymers (e.g., polyisobutylene-polystyrene block copolymers such as SIBS), polyvinyl ketones, polyvinylcarbazoles, and polyvinyl esters such as polyvinyl acetates; polybenzimidazoles; ionomers; polyalkyl oxide polymers and copolymers including polyethylene oxides (PEO); glycosaminoglycans; polyesters including polyethylene terephthalates and aliphatic polyesters such as polymers and copolymers of lactide (which includes lactic acid as well as d-,l- and meso lactide), epsilon-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, and 6,6-dimethyl-1,4-dioxan-2-one (a copolymer of polylactic acid and polycaprolactone is one specific example); polyether polymers and copolymers including polyarylethers such as polyphenylene ethers, polyether ketones, polyether ether ketones; polyphenylene sulfides; polyisocyanates; polyolefin polymers and copolymers, including polyalkylenes such as polypropylenes, polyethylenes (low and high density, low and high molecular weight), polybutylenes (such as polybut-1-ene and polyisobutylene), polyolefin elastomers (e.g., santoprene), EPDM (ethylene propylene diene monomer) rubbers, poly-4-methyl-pen-1-enes, ethylene-alpha-olefin copolymers, ethylene-methyl methacrylate copolymers and ethylene-vinyl acetate copolymers; fluorinated polymers and copolymers, including polytetrafluoroethylenes (PTFE), poly(tetrafluoroethylene-co-hexafluoropropene) (FEP), modified ethylene-tetrafluoroethylene copolymers (ETFE), and polyvinylidene fluorides (PVDF); silicone polymers and copolymers; polyurethanes; p-xylylene polymers; polyiminocarbonates; copoly(ether-esters)such as polyethylene oxide-polylactic acid copolymers; polyphosphazines; polyalkylene oxalates; polyoxaamides and polyoxaesters (including those containing amines and/or amido groups); polyorthoesters; biopolymers, such as polypeptides, proteins, polysaccharides and fatty acids (and esters thereof), including fibrin, fibrinogen, collagen, elastin, chitosan, gelatin, starch, glycosaminoglycans such as hyaluronic acid; as well as blends and copolymers of the above.

Such polymers may be provided in a variety of configurations, including cyclic, linear and branched configurations. Branched configurations include star-shaped configurations (e.g., configurations in which three or more chains emanate from a single branch point), comb configurations (e.g., graft polymers having a main chain and a plurality of branching side chains), and dendritic configurations (e.g., arborescent and hyperbranched polymers). The polymers can be formed from a single monomer (i.e., they can be homopolymers), or they can be formed from multiple monomers (i.e., they can be copolymers) that can be distributed, for example, randomly, in an orderly fashion (e.g., in an alternating fashion), or in blocks.
Biologically active agents are loaded in accordance with the present invention for any number of purposes, for example, to effect *in vivo* release (which may be, for example, immediate or sustained) of the biologically active agents, to affect tissue adhesion vis-à-vis the medical device, to influence thromboresistance, to influence antihyperplastic behavior, to enhance recellularizaton, and to promote tissue neogenesis, among many other purposes.

"Biologically active agents," "drugs," "therapeutic agents," "pharmaceutically active agents," "pharmaceutically active materials," and other related terms may be used interchangeably herein and include genetic biologically active agents, non-genetic biologically active agents and cells. Biologically active agents may be used singly or in combination.

Exemplary non-genetic biologically active agents for use in connection with the present invention include: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (l) antimicrobial agents such as triclosan, cephalosporins, antimicrobial peptides such as magainins, aminoglycosides and nitrofurantoin; (m) cytotoxic agents, cytostatic agents and cell proliferation affectors; (n) vasodilating agents; (o)agents that interfere with endogenous vasoactive mechanisms, and

(p) inhibitors of leukocyte recruitment, such as monoclonal antibodies. Preferred non-genetic biologically active agents include paclitaxel, sirolimus, everolimus, tacrolimus, dexamethasone, estradiol, ABT-578 (Abbott Laboratories), trapidil, liprostin, Actinomcin D, Resten-NG, Ap-17, abciximab, clopidogrel and Ridogrel.

Exemplary genetic biologically active agents for use in connection with the present invention include anti-sense DNA and RNA as well as DNA coding for: (a) anti-sense RNA, (b) tRNA or rRNA to replace defective or deficient endogenous molecules, (c) angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic biologically active agents include (a) plasmids, (b) viral vectors such as adenovirus, adenoassociated virus and lentivirus, and (c) non-viral vectors such as lipids, liposomes and cationic lipids.

Cells for use in connection with the present invention include cells of human origin (autologous or allogeneic), including stem cells wherein the cells are not human embryonic cells or from an animal source (xenogeneic), which can be genetically engineered, if desired, to deliver proteins of interest.

Numerous biologically active agents, not necessarily exclusive of those listed above, have been identified as candidates for vascular treatment regimens, for example, as agents targeting restenosis. Such agents are useful for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) ACE inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (l) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate , nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, paclitaxel and epothilone), caspase activators, proteasome inhibitors, angiogenesis inhibitors (e.g., endostatin, angiostatin and squalamine), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Numerous additional biologically active agents, not necessarily exclusive of those listed above, are also disclosed in U.S. Patent No. 5,733,925 assigned to NeoRx Corporation.

Numerous other biologically active agents, not necessarily exclusive of those listed above, have been identified as candidates for influencing tissue adhesion to medical devices. Examples include proteoglycans and glycosaminoglycans (GAGs), for instance, hyaluronic acid (e.g., to inhibit tissue adhesion), keratan, perlecan, dermatin, heparin and chondroitin, as well as various salts of the same, such as hyaluronates, dermatin sulfates, heparin sulfates, keratan sulfates and chondroitin sulfates; cell adhesion peptides (e.g., RGD peptides); adhesive proteins (e.g., fibronectin, laminin, vitronectin, etc.); and growth factors. Synthetic materials also can be used to control biologic reactions and can have biologic activity as well. For example, sulfonated polymers can act as synthetic heparinoids, and synthetic hydrogels (e.g., PEG) can act as anti-adhesives.

A range of drug loading levels can be used in connection with the various embodiments of the present invention, with the amount of loading being readily determined by those of ordinary skill in the art and ultimately depending, for example, upon the condition to be treated, the nature of the biologically active agent itself, the means by which the biologically active agent is administered to the intended subject, and so forth.

## Claims

1. A method of loading a medical article with a biologically active agent, said method comprising:
providing a tubular medical article, said medical article comprising a nanoporous surface region on its abluminal surface and its luminal surface; and
contacting said abluminal nanoporous surface region of said medical article with a supercritical fluid comprising a carrier fluid and a first biologically active agent and contacting said luminal nanoporous surface region of said medical article with a supercritical fluid comprising a carrier fluid and a second biologically active agent different from the first biologically active agent.

2. The method of claim 1 wherein the carrier fluid is carbon dioxide.

3. The method of claim 1, wherein said biologically active agent is dissolved or colloidally suspended in the supercritical fluid.

4. The method of claim 1,
wherein said biologically active agent is an antirestenotic agent, preferably is paclitaxel, or
wherein said biologically active agent is an agent that promotes tissue adhesion, or
wherein said biologically active agent is selected from glycosaminoglycans, proteoglycans, cell adhesion peptides and adhesive proteins, or
wherein said biologically active agent is selected from hyaluronic acid, dermatin, perlecan, heparin, keratan, chondroitin and salts of the same.

5. The method of claim 1, wherein said nanoporous surface region comprises a metal, in particular a noble metal, preferably a metal alloy selected from stainless steel alloys, cobalt-chromium-iron alloys, nickel-chromium alloys, inconel alloys, cobalt-chromium alloys, and nickel-titanium alloys.

6. The method of claim 1,
wherein the nanoporous surface region comprises a bioactive oxide, or
wherein the nanoporous surface region comprises an oxide selected from aluminum oxides, silicon oxides, alkaline earth metal oxides and transition metal oxides, or
wherein the nanoporous surface region comprises hydroxyapatite.

7. The method of claim 1, wherein the nanoporous surface region comprises a polymer, wherein said polymer is preferably selected from acrylate polymers and copolymers, methacrylate polymers and copolymers, polyimide polymers and copolymers, polysulfone polymers and copolymers, polyamide polymers and copolymers, polymers and copolymers of vinyl monomers, polyolefin polymers and copolymers, fluorinated polymers and copolymers, silicone polymers and copolymers, and polyurethanes.

8. The method of claim 1, wherein said medical article is an implantable or insertable medical device.

9. The method of claim 8, wherein said medical device is selected from bone plates, joint prostheses, vascular grafts, stent grafts, stents, catheters, guide wires, balloons, filters, vascular patches, shunts, and coils.

10. A medical device obtainable by the method of claim 1.

## Patentansprüche

1. Verfahren zum Beladen eines medizinischen Artikels mit einem biologisch aktiven Wirkstoff, wobei das Verfahren aufweist:
Bereitstellen eines röhrenförmigen medizinischen Artikels, wobei der medizinische Artikel einen nanoporösen Oberflächenbereich auf seiner abluminalen Oberfläche und seiner luminalen Oberfläche aufweist; und
In-Kontakt-bringen des abluminalen nanoporösen Oberflächenbereichs des medizinischen Artikels mit einem überkritischen Fluid, das ein Trägerfluid und einen ersten biologisch aktiven Wirkstoff aufweist, und In-Kontakt-bringen des luminalen nanoporösen Oberflächenbereichs des medizinischen Artikels mit einem überkritischen Fluid, das ein Trägerfluid und einen zweiten biologisch aktiven Wirkstoff aufweist, der sich vom ersten biologisch aktiven Wirkstoff unterscheidet.

2. Verfahren nach Anspruch 1, wobei das Trägerfluid Kohlendioxid ist.

3. Verfahren nach Anspruch 1, wobei der biologisch aktive Wirkstoff im überkritischen Fluid gelöst oder kolloidal suspendiert ist.

4. Verfahren nach Anspruch 1,
wobei der biologisch aktive Wirkstoff ein Antirestenosewirkstoff, vorzugsweise Paclitaxel, ist oder
wobei der biologisch aktive Wirkstoff ein Wirkstoff ist, der Gewebsadhäsion fördert, oder
wobei der biologisch aktive Wirkstoff aus Glykosaminoglykanen, Proteoglykanen, Zelladhäsionspeptiden und adhäsiven Proteinen ausgewählt ist oder
wobei der biologisch aktive Wirkstoff aus Hyaluronsäure, Dermatin, Perlecan, Heparin, Keratan, Chondroitin und deren Salzen ausgewählt ist.

5. Verfahren nach Anspruch 1, wobei der nanoporöse Oberflächenbereich ein Metall, insbesondere ein Edelmetall, vorzugsweise eine Metalllegierung aufweist, die aus Edelstahllegierungen, Cobalt-Chrom-Eisen-Legierungen, Nickel-Chrom-Legierungen, Inconel-Legierungen, Cobalt-Chrom-Legierungen und Nickel-Titan-Legierungen ausgewählt ist.

6. Verfahren nach Anspruch 1,
wobei der nanoporöse Oberflächenbereich ein bioaktives Oxid aufweist oder
wobei der nanoporöse Oberflächenbereich ein Oxid aufweist, das aus Aluminiumoxiden, Siliciumoxiden, Erdalkalimetalloxiden und Übergangsmetalloxiden ausgewählt ist, oder
wobei der nanoporöse Oberflächenbereich Hydroxyapatit aufweist.

7. Verfahren nach Anspruch 1, wobei der nanoporöse Oberflächenbereich ein Polymer aufweist, wobei das Polymer vorzugsweise aus Acrylatpolymeren und -copolymeren, Methacrylatpolymeren und -copolymeren, Polyimidpolymeren und -copolymeren, Polysulfonpolymeren und -copolymeren, Polyamidpolymeren und - copolymeren, Polymeren und Copolymeren von Vinylmonomeren, Polyolefinpolymeren und -copolymeren, Fluorpolymeren und -copolymeren, Silikonpolymeren und -copolymeren und Polyurethanen ausgewählt ist.

8. Verfahren nach Anspruch 1, wobei der medizinische Artikel ein implantierbares oder einführbares Medizinprodukt ist.

9. Verfahren nach Anspruch 8, wobei das Medizinprodukt aus Knochenplatten, Gelenkprothesen, Gefäßtransplantaten, Stenttransplantaten, Stents, Kathetern, Führungsdrähten, Ballons, Filtern, Gefäßpatches, Shunts und Coils ausgewählt ist.

10. Medizinprodukt, das durch das Verfahren nach Anspruch 1 erhältlich ist.

## Revendications

1. Procédé de chargement d'un article médical avec un agent biologiquement actif, ledit procédé comprenant :
la préparation d'un article médical tubulaire, ledit article médical comportant une zone de surface nanoporeuse sur sa surface abluminale et sa surface luminale ; et
la mise en contact de la zone de surface nanoporeuse abluminale de l'article médical avec un fluide supercritique comportant un fluide porteur et un premier agent biologiquement actif, et la mise en contact de la zone de surface nanoporeuse luminale de l'article médical avec un fluide supercritique comportant un fluide porteur et un deuxième agent biologiquement actif différent du premier agent biologiquement actif.

2. Procédé selon la revendication 1 où le fluide porteur est le dioxyde de carbone.

3. Procédé selon la revendication 1, où l'agent biologiquement actif est présent dissous ou en suspension colloïdale dans le fluide supercritique.

4. Procédé selon la revendication 1,
où l'agent biologiquement actif est un agent antiresténotique, préférentiellement le paclitaxel, ou bien
où l'agent biologiquement actif est un agent promoteur de l'adhérence tissulaire, ou bien
où l'agent biologiquement actif est sélectionné entre glycosaminoglycanes, protéoglycanes, peptides d'adhérence cellulaire et protéines adhésives, ou bien où l'agent biologiquement actif est sélectionnée entre acide hyaluronique, dermatin, perlécane, héparine, kératane, chondroïtine et sels correspondants.

5. Procédé selon la revendication 1, où la zone de surface nanoporeuse comprend un métal, en particulier un métal noble, préférentiellement un alliage métallique sélectionné entre alliages d'acier inoxydable, alliages cobalt-chrome-fer, alliages nickel-chrome, alliages Inconel, alliages cobalt-chrome et alliages nickel-titane.

6. Procédé selon la revendication 1,
où la zone de surface nanoporeuse comprend un oxyde bioactif, ou bien où la zone de surface nanoporeuse comprend un oxyde sélectionné entre oxydes d'aluminium, oxydes de silicium, oxydes de métaux alcalino-terreux et oxydes de métaux transitoires, ou bien
où la zone de surface nanoporeuse comprend de l'hydroxyapatite.

7. Procédé selon la revendication 1, où la zone de surface nanoporeuse comprend un polymère, ledit polymère étant préférentiellement sélectionné entre polymères et copolymères acrylates, polymères et copolymères méthacrylates, polymères et copolymères polyimides, polymères et copolymères polysulfones, polymères et copolymères polyamides, polymères et copolymères de vinyles monomères, polymères et copolymères polyoléfines, polymères et copolymères fluorés, polymères et copolymères de silicone et polyuréthanes.

8. Procédé selon la revendication 1, où l'article médical est un dispositif médical implantable ou insérable.

9. Procédé selon la revendication 8, où le dispositif médical est sélectionné entre plaques d'ostéosynthèse, prothèses articulaires, greffes vasculaires, greffes d'endoprothèses, stents, cathéters, fils guides, ballonnets, filtres, pièces vasculaires, shunts et bobines.

10. Dispositif médical obtenu au moyen du procédé selon la revendication 1.
